(19) Europäisches Patentamt
European Patent Office
Office européen des brevets

(11) Publication number : **0 485 173 A2**

(12) # EUROPEAN PATENT APPLICATION

(21) Application number : **91310226.5**

(22) Date of filing : **05.11.91**

(51) Int. Cl.$^5$ : **C08B 37/08,** C08F 8/00, C08G 73/04, A61K 47/48

(30) Priority : **06.11.90 US 609881**
**11.10.91 US 776888**

(43) Date of publication of application :
**13.05.92 Bulletin 92/20**

(84) Designated Contracting States :
**BE CH DE ES FR GB IT LI NL SE**

(71) Applicant : **FGN, INC.**
**Ventana Canyon Center, 5620 North Kolb Road**
**Tucson, Arizona 85715 (US)**

(71) Applicant : **THE UNIVERSITY OF ARIZONA**
**Tucson, Arizona 85721 (US)**

(72) Inventor : **Pamukcu, Rifat**
**1224 Herschel**
**Cincinatti, Ohio 45208 (US)**
Inventor : **Gross, Paul**
**126 West McKenzie**
**Stockton, California 95204 (US)**
Inventor : **Brendel, Klaus**
**3231 North Manor Drive**
**Tucson, Arizona 85715 (US)**

(74) Representative : **Baverstock, Michael George Douglas et al**
**BOULT, WADE & TENNANT 27 Furnival Street**
**London, EC4A 1PQ (GB)**

(54) **Esters and amides of substituted fused ring phenyl acetic acids.**

(57) Esters and amides of substituted fused ring phenyl acetic acids having the formula

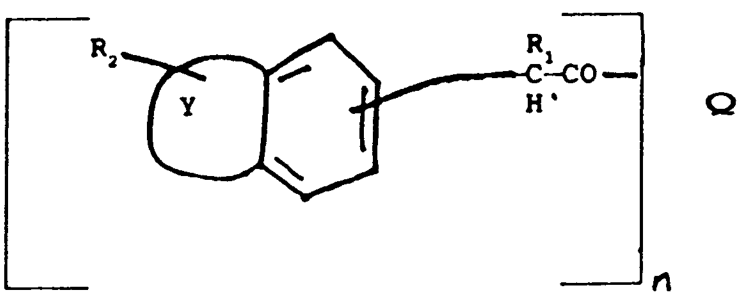

wherein Y, together with the ring to which it is fused forms a multi-ring system selected from napthyl, [1]benzopyranol [2, 3-b], pyridine, phenothiazine, carbazole, and benzoxazole ;

$R_1$ is lower alkyl or hydrogen ;

$R_2$ is one or more independently selected from cyano, nitro, amino, alkylamino, hydrogen, halogen, lower alkyl, lower alkoxy, hydroxy, phenyl or phenyl substituted with halogen, alkyl or alkoxy ;

wherein Q is the deprotonated residue of a polymer or macromolecular structure having a molecular weight of at least 1000 containing at least two primary and/or secondary amino groups and/or hydroxy groups ; and

n is an integer of at least 2 are useful in treating colonic polyps.

EP 0 485 173 A2

This invention relates to compounds and compositions for use in the treatment or prevention of colonic polyps.

Each year in the United States alone, approximately 60,000 people die from colon cancer, and over 150,000 new cases of colon cancer are diagnosed. For the American population as a whole, individuals have a six percent lifetime risk of developing colon cancer, making it the second most prevalent form of cancer in the country. Colon cancer is also prevalent in Western Europe.

To date, little progress has been made in the prevention and treatment of colorectal cancer, as reflected by the lack of change in the five-year survival rate over the last few decades. The only cure for this cancer is surgery at an extremely early stage. Unfortunately, most of these cancers are discovered too late for surgical cure, because most victims do not experience symptoms until the disease is advanced.

The incidence of colon cancer increases with age, particularly after the age of 40. Since the mean ages of populations in America and Western Europe are increasing, the prevalence of colorectal cancer should increase in the future.

In view of these grim statistics, efforts in recent years have concentrated on colon cancer prevention. Colon cancer usually arises from pre-existing benign growths known as polyps. Prevention efforts have emphasized the identification and removal of colonic polyps. Polyps are identified by x-ray and/or colonoscopy, and usually removed by devices associated with the colonoscope. The increased use of colon x-rays and colonoscopies in recent years has detected clinically significant precancerous polyps in four to six times the number of individuals per year that acquire colon cancer. During the past five years alone, an estimated 3.5 to 5.5 million people in the United States have been diagnosed with adenomatous colonic polyps, and it is estimated that many more people have or are susceptible to developing this condition, but are as yet undiagnosed. In fact, there are estimates that 10-12 percent of people over the age of 40 will form clinically significant adenomatous polyps.

Removal of polyps has been accomplished either with surgery or fiber-optic endoscopic polypectomy -- procedures that are uncomfortable, costly (the cost of a single polypectomy ranges between $1,000 and $1,500 for endoscopic treatment and more for surgery), and involve a small but significant risk of colonic perforation. Overall, about $2.5 billion is spent annually in the United States in colon cancer treatment and prevention.

As indicated above, each polyp carries with it a chance that it will develop into a cancer. The likelihood of cancer is diminished if a polyp is removed. However, many of these patients demonstrate a propensity for developing additional polyps in the future. They must, therefore, be monitored periodically for the rest of their lives for polyp reoccurrence.

In most cases (i.e. the cases of so-called common sporadic polyps), polyp removal will be effective to reduce the risk of cancer. In a small percentage of cases (i.e. the cases of the so-called polyposis syndromes), removal of all or part of the colon is indicated. The difference between common sporadic polyps and polyposis syndromes is dramatic. Common sporadic polyp cases are characterized by relatively few polyps, each of which can usually be removed leaving the colon intact. By contrast, polyposis syndrome cases can be characterized by many (e.g. hundreds or more) of polyps -- literally covering the colon in some cases, making safe removal of the polyps impossible short of surgical removal of the colon. Because each polyp carries with it the palpable risk of cancerous development, polyposis syndrome patients invariably develop cancer if left untreated. Many of these patients have undergone a severe change in lifestyle as a result of surgery. Patients have strict dietary restrictions, and many must wear ostomy appliances to collect their intestinal wastes.

Recently, several non-steroidal anti-inflammatory drugs ("NSAIDs"), originally developed to treat arthritis, have shown effectiveness in inhibiting and eliminating polyps. Colonic polyps virtually disappear when the patient take the drug. However, the prophylactic use of currently available NSAIDs, even in polyposis syndrome patients, is marked by severe side reactions that include gastrointestinal irritations and ulcerations. Once NSAID treatment is terminated due to such complications, the polyps return, particularly in polyposis syndrome patients.

This invention provides a novel class of compounds of formula I below that are effective in eliminating and inhibiting polyps, but are not characterized by the severe side reactions of NSAIDs.

This invention also enables a method of treating patients with common sporadic polyps and polyposis syndromes to reduce or eliminate their polyps by administering to a patient in need of such treatment a physiologically effective amount of a compound of formula I.

As discussed above, the present invention provides a class of compounds of formula I below:

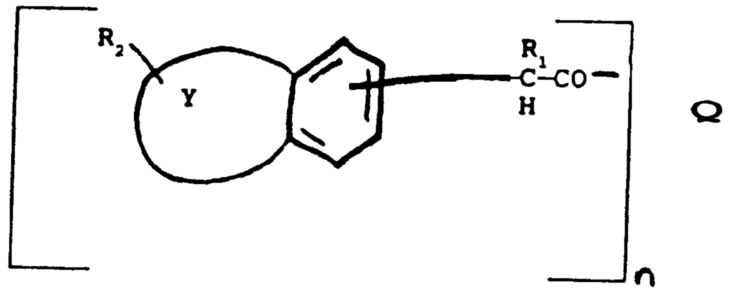

I

wherein Y, together with the ring to which it is fused forms a multi-ring system selected from napthyl, [1]benzopyranol [2, 3-b], pyridine, phenothiazine, carbazole, and benzoxazole;

$R_1$ is lower alkyl or hydrogen;

$R_2$ is one or more independently selected from cyano, nitro, amino, alkylamino, hydrogen, halogen, lower alkyl, lower alkoxy, hydroxy, phenyl or phenyl substituted with halogen, alkyl or alkoxy;

wherein Q is the deprotonated residue of a polymer or macromolecular structure having a molecular weight of at least 1000 containing at least two primary and/or secondary amino groups and/or hydroxy groups; and

n is an integer of at least 2.

As used herein, the term "halo" or "halogen" refers to chloro, bromo, fluoro and iodo groups, and the term "alkyl" or "alkoxy" refers to straight, branched or cyclic groups. The term "lower alkyl" or "lower alkoxy" refers to groups containing from 1-5 carbon atoms.

As used herein, the term macromolecule, macromolecular structure, or polymer refers to molecules having at least two primary and/or secondary amino groups, and/or hydroxy groups. Examples of such amino-containing polymers or macromolecules are polyvinylamine, polyallylamine, polyethyleneimine, chitosan, polyamino acids, polyamine exchange resins (e.g. Amberlite) and polyaminoalkanes. Examples of hydroxy-containing polymers or macromolecules are polyhydroxyalkanes, polyvinylalcohols, carbohydrates (e.g. sucrose), and polyethylene glycols. The term "deprotonated residue" includes the situation where at least some, but not all, of the amino and/or hydroxy groups are deprotonated on the macromolecule or polymer.

Compounds of this invention have unexpected utility for suppression of colonic polyps in view of the startling discovery that the effects of conventional NSAID therapy on colonic polyps can be, in fact, achieved via topical exposure to the agents. This effect was discovered in a patient with familial polyposis, a disease characterized by the presence of numerous colonic polyps. In an attempt to avoid colon cancer, the patient underwent surgical excision of the colon with formation of a continent ileostomy, or Kock's pouch. By this rarely performed surgical procedure, a pouch is constructed from the terminal portion of the small intestine. A colonic bacterial environment developed within the pouch resulting in extensive adenomatous polyp formation. Polyps also developed on the stoma, an external outlet from the pouch constructed from a contiguous portion of small intestine, and in the duodenum, the beginning of the small intestine. One NSAID, when administered in oral doses, led to the disappearance of the numerous polyps located in the pouch but not the polyps on the stoma or in the duodenum. Given the understanding of the metabolic and excretory patterns of the drug as well as of bacterial enzyme activation of the agent, these rare findings suggested that high local concentrations of the drug were responsible for the effects in the pouch. It was apparent from the lack of response of the polyps in the other locations, particularly the stomal polyps which are close to the pouch, that the effect was topical and that blood-borne or systemic delivery of the drug was ineffectual.

The topical effect is particularly surprising since the cells believed to be responsible for polyp growth and subsequent malignancy are not only epithelial cells deep within the crypts of the intestine, but may also include cells which modulate the local immunological defense mechanisms in deeper mucosal and serosal layers of the intestinal wall.

Compounds of this invention deliver active agents to the colon via the large macromolecular structure to which the active agent is conjugated. Colonic bacterial enzymes (or other colonic enzymes) cleave the active agent from the macromolecule, achieving locally high concentrations of the active agent and allowing the agent to contact the colon itself leading to inhibition of polyp proliferation.

The advantage of this treatment is that the active agent can be concentrated where it is effective, but whatever systemic levels are achieved are minimized. The systemic levels are particularly low because only passive absorption in the colon is involved. The negligible systemic levels are important in that the maintenance of

chronic systemic levels of NSAIDs is complicated by a high incidence of gastric ulcers rendering them useless in a long-term prophylactic regimen.

Thus, contrary to prior approaches that relied on the high systemic levels of active agent to achieve the desired effect within the colon with the consequent gastric complications, the compounds of the present invention afford a different and safer therapeutic approach in light of the topical effect of these active agents on the colon itself.

The present invention enables a method for treating patients having colonic polyps to reduce the polyps which involves administering to the patient a therapeutically effective amount of a compound of formula I where $R_1$, $R_2$, Y and n are as defined above, but Q is the deprotonated residue of a polyamine or polyhydroxy compound.

Compounds of Formula I may be formulated into compositions together with pharmaceutically acceptable carriers for parenteral injection, for oral administration in solid or liquid form, for rectal administration, and the like, although oral administration is most preferred.

Compositions according to the present invention for parenteral injection may comprise pharmaceutically acceptable sterile aqueous or nonaqueous solutions, suspensions or emulsions. Examples of suitable nonaqueous carriers, diluents, solvents or vehicles include propylene glycol, vegetable oils, such as olive oil, and injectable organic esters such as ethyl oleate. Such compositions may also contain adjuvants such as preserving, wetting, emulsifying and dispersing agents. They may be sterilized, for example, by filtration through a bacteria-retaining filter, by incorporating sterilizing agents into the compositions, or irradiation. They can also be manufactured in the form of sterile solid compositions which can be dissolved in sterile water, or some other sterile injectable medium immediately before use.

Solid dosage forms for oral administration include capsules, tablets, pills, powders, troches and granules. In such solid dosage forms, the active compound is admixed with at least one inert diluent such as sucrose, lactose or starch. Such dosage forms can also comprise, as is normal practice, additional substances other than diluents, e.g., lubricating agents such as magnesium stearate. In the case of capsules, tablets, troches and pills, the dosage forms may also comprise buffering agents. Tablets, pills and granules can additionally be prepared with enteric coatings.

Liquid dosage forms for oral administration include pharmaceutically acceptable emulsions, solutions, suspensions, syrups and elixirs containing inert diluents commonly used in the art, such as water. Besides such inert diluents, compositions can also include adjuvants such as wetting agents, emulsifying and suspending agents, and sweetening, flavoring and perfuming agents.

Compositions for rectal administration are preferably suppositories which may contain, in addition to the active substance, excipients such as cocoa butter or a suppository wax.

Actual dosage levels of active ingredient in the compositions of the invention may be varied so as to obtain an amount of active ingredient effective to achieve polyp-eliminating activity in accordance with the desired method of administration. The selected dosage level therefore depends upon the nature of the active compound administered, the route of administration, the desired duration of treatment, and other factors. If desired, the daily dose may be divided into multiple doses for administration, e.g. two to four times per day.

Compounds of this invention can be made by one of the five general schemes below.

SCHEME I

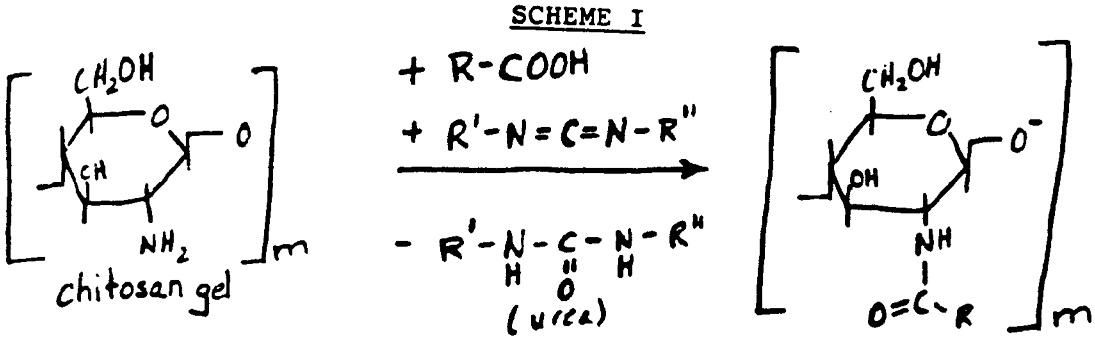


This scheme is useful for cases, where Q is water swellable polymer carrying aminogroups. The water soluble carbodiimide allows acylation in the alcoholic-aqueous phase, and the water soluble by-product urea can be removed by water, from the acylated polymer. The scheme allows acylation with carboxylic acid sensitive to the conditions of acid chloride or acid anhydride formation.

A chitosan gel $(GlcN)_m$ is prepared from chitosan, according to the method of S. Hirano et al. (Carbohyd.

Res. 201 (1990) pp. 145-149) where m is the number of repeating units within the chitosan molecule. The gel is stirred in 70% aqueous methanol solution, at 0°-5° C, with the R-carboxylic acid (2 equivalents per GlcN; where R is the group in the brackets in formula I minus the attached carbonyl), and with a water-soluble carbodiimide (R′-N=C=N-R″; 2 equivalents per GlcN) for three days. (R′ and R″ are cycloalkyl or alkyl and the like, containing also quaternary ammonium or sulfonate salt for solubilization of the carbodiimide) The resulting gel is homogenized, washed well with distilled water, stirred with NaOH (1.2 equivalents per GlcN) in water (50 ml per g of chitosan) for five days. The mixture is homogenized and washed to neutrality. The gel is then dried to an amorphous powder.

SCHEME II

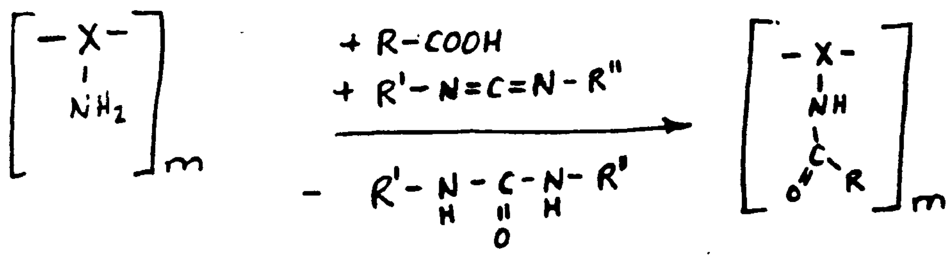

This scheme is useful for cases, where a salt between R-COOH and a polyamine Q is swellable or soluble in DMF. The carbodiimide is chosen, so that the by-product urea is soluble in dichloromethane, and therefore removable by extraction with it. Sodium hydroxide is used to extract unreacted R-COOH, so this scheme is useful for cases where acylation is difficult and incomplete. The scheme especially allows acylation with carboxylic acids that degrade under the conditions of acid chloride or acid anhydride formation.

$(GlcN)_m$ "chitosan," polylysine or similar polyamine "X" (0.01 mol-$NH_2$ groups; where m is the number of repeating amino-containing units per molecule of polyamine) are rapidly stirred in dimethylformanide ("DMF," 30 ml) at 50°C until no further dissolution is apparent. The cooled (0°) mixture is treated with carbodiimide (R′-N=C=N-R"; 0.011 mol) with continued stirring for two days. The resultant solution or suspension is poured into ice water. The precipitate is filtered off and washed with water. It is purified by being homogenized with and filtered from (a) $CH_2Cl_2$ (2x 50 ml); (b) 0.1 N-NaOH (2 x 50 ml); (c) 0.1N-HCl (2 x 50 ml); (d) $H_2O$ (2 x 50 ml); and (e) ether (2 x 50

SCHEME III

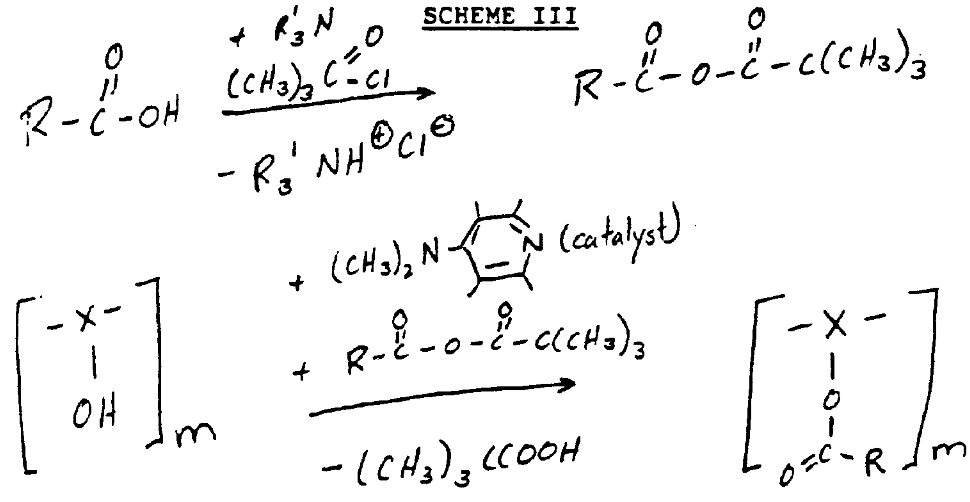


This scheme is suitable for cases were q is a hydroxyl group-containing polymer that is soluble or swellable in dimethyl formamide. The bulky t-butyl group in pivalic acid prevents acylation by it, and dimethylamino pyridine catalyzes the difficult O-acylation. By-product pivalic acid is removable from the acylated polymer by extraction with organic solvent (e.g. toluene). The scheme is useful for carboxylic acids that are sensitive to the conditions of acylchloride synthesis.

Dry polyvinyl alcohol, methyl cellulose, or a similar swellable carbohydrate ($[X\text{-}OH]_m$; 0.01 mol-OH; where

"m" is the number of repeating hydroxy-containing units in the polymeric compound) is rapidly stirred in absolute dimethyl formamide ("DMF," 50 ml) at 50°C until no further dissolution is apparent. Separately the carboxylic acid (RCOOH; 0.01 mol) is dissolved in absolute tetrahydrofuran (30 ml). At -10°C, pivaloyl chloride (0.01 mol) is added, followed by drop wise addition of a tertiary amine ($R'_3N$) (0.01 mol, e.g., triethylamine, ethyl diisopropylamine). The precipitated amine hydrochloride is filtered off. The solution is added, drop by drop, to the stirred and cooled (-10°C) polyol or carbohydrate mixture. The combined mixture is treated at -10°C with p-dimethyl amino pyridine (0.0001 mol.), and is allowed to come to room temperature and stay there for 15 hours. Toluene (100 ml) is added, with stirring. The mixture is evaporated to dryness in a rotary evaporator. The residue is homogenized in and filtered from (a) toluene (100 ml) and (b) water (2 x 100 ml). The filtercake is dried in vacuo at 40°C to constant weight.

SCHEME IV

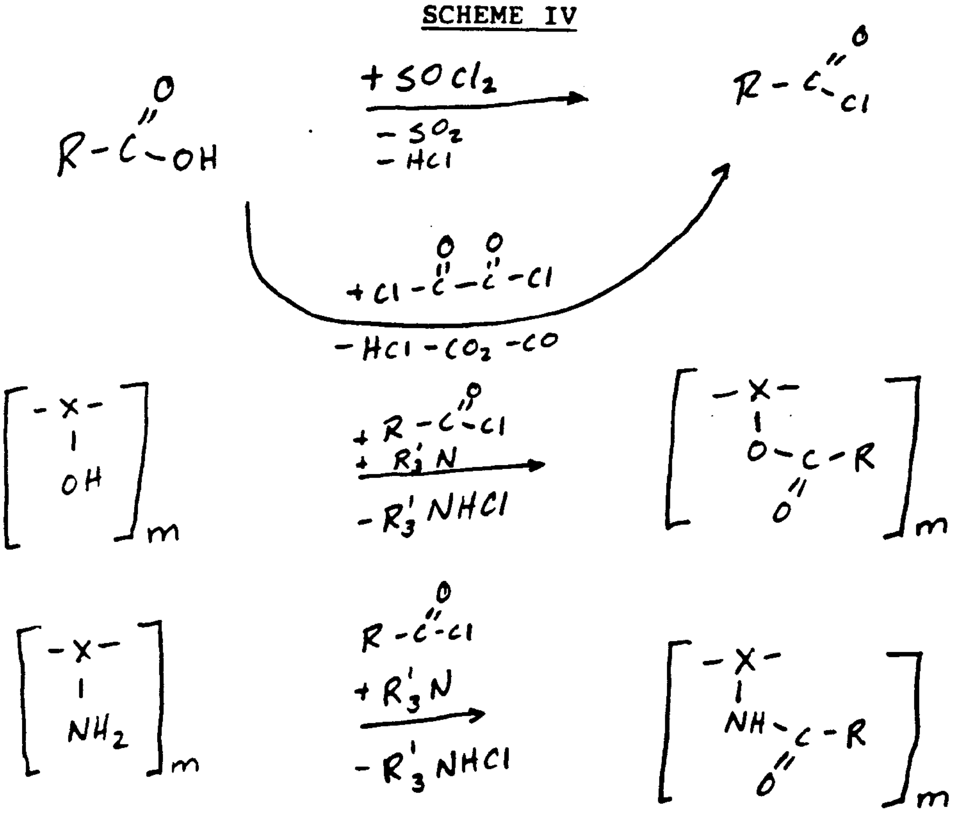


This scheme is useful for cases where Q is a polymer swellable in dimethyl formamide (DMF), and where it needs a highly reactive reagent for acylation. The scheme is suitable for carboxylic acids that form stable acid chlorides.

Carboxylic acid (R-COOH; 0.01 mol) is refluxed with thionylchloride or oxalylchloride (20 ml) until solution is complete and gas evolution ceases. Excess reagent is removed by evaporation. The residual acid chloride is diluted with tetrahydrofuran (10 ml) to give solution A.

A polyamine ($[-X-NH_2]_m$) such as chitosan, aminoethyl cellulose, polylysine (0.01 mol-$NH_2$), or a polyhydroxy compound ($[-X-OH]_m$) such as polyvinyl alcohol or a carbohydrate (e.g. methyl cellulose; 0.01 mol-OH) are heated in absolute dimethyl formamide at 50°C, until no further dissolution is apparent. Pyridine (0.01 mol) and p-dimethylaminopyridine (0.01 mol) are added. The mixture is cooled to -10°C, and solution A is added slowly with stirring. After 15 hours at room temperature, toluene (100 mol) is added, and the solution is evaporated in vacuo. The residue is homogenized with and filtered from (a) water (2 x 100 ml); (b) ether (2 x 100 ml), and is dried.

SCHEME V

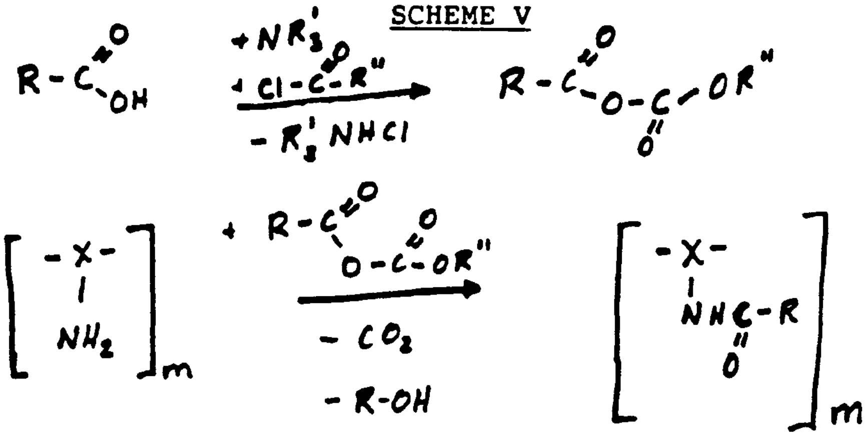


This scheme is useful, where Q is a polyamine swellable or soluble in dimethylformamide. It is especially suitable for cases where the removal of by-products such as salts, acids, or bases) from the final product is difficult, since in this case only carbon dioxide and low molecular weight alcohol are produced as by-products. The scheme is especially useful for carboxylic acids that decompose under the conditions of acid chloride synthesis.

Chitosan, amino ethyl cellulose, polylysine or a similar polyamine ($[-X-NH_2]_m$; 0.01 mol-$NH_2$ groups) is rapidly stirred in dimethyl formamide (30 ml) at 50°C until no further dissolution is apparent. The carboxylic acid (RCOOH; 0.01 mol) is dissolved in absolute tetrahydrofuran (30 ml). At first trialkylamine ($NR'_3$; 0.01 mol), and then alkylchlorocarbonate (Cl-COOR″; 0.01 mol, where R″ is ethyl or isobutyl) is added. The precipitated trialkylammonium chloride ($R'_3NHCl$) is filtered off. The filtrate is added, with stirring, to the cold (-30°C) polyamine solution. After being stored for 15 hours at -15°C, the mixture is poured on ice (300 g), with stirring. After the ice has melted, the precipitate is filtered off, is thoroughly washed with water, and is dried.

The foregoing may be better understood from the following examples, which are presented for purposes of illustration and are not intended to limit the scope of the invention. As used in the following examples, the references to compounds such as (1), (2), (3) etc., and to substituents such as R, $R_1$, $R_2$ etc., refer to the corresponding compounds and substituents in the foregoing reaction schemes and in formula I.

## EXAMPLE 1

### Metiazinic Acid Amide Of Chitosan

Metiazinic Acid is conjugated to chitosan gel (0.01 mol-$NH_2$) according to Scheme I. The procedure yields the desired compound ($R_1$ = hydrogen, $R_2$ = $CH_3$; Y = phenothiazine; Q = chitosan; m > 50; n/m > 0.2; n > 10).

## EXAMPLE 2

### Polyvinyl Alcohol Ester of Naproxen

A) Naproxen acid chloride

Thionyl chloride 4.35 ml (0.059 mol) was added to a suspended solution of 12.5 g (0.054 mol) naproxen in 60 ml of toluene. The reaction mixture was refluxed under nitrogen for 60 minutes. The solvent was removed in vacuo to give naproxen acid chloride as a white powder. IR (Nujor mull) 2954, 2923, 1790, 1604, 1460, 1377, 1229, 1026 $cm^{-1}$.

B) Polyvinyl alcohol ester of naproxen

Polyvinyl alcohol (average molecular weight: 50,000) 2.39 g was added to 100 ml of pyridine. To this suspended solution was added 6.7 g (0.27 mol) of naproxen acid chloride. The reaction mixture was slowly heated until all PVA dissolved, and then refluxed for one hour. The clear solution was concentrated in vacuo then washed with 200 ml of 1 N NaOH solution. The aqueous part was decanted, and the residue oil was washed with five 200 ml portions of water to remove pyridine and unreacted PVA. The product was dried over phosphorus pentoxide ($P_2O_5$) in a vacuum desiccator at room temperature for 12 days. It gave 4.2 g of the polyvinyl

alcohol ester of naproxen as a yellow solid.

IR (film) 3453, 2927, 2852, 1729, 1606, 1264, 1218 $cm^{-1}$.

Elemental analysis calculated for $C_{18}H_{20}O_4$; FW 300; % Theory: C, 72.00; H, 6.66; % Found : C, 71.77; H, 7.10.

The procedure yields the desired compound $R_1$ = methyl; $R_2$ = 6-methoxy; Y = naphthyl; Q = polyvinyl alcohol; n/m = 0.5.

EXAMPLE 3

Poly ε-[Pranoprofenyl] Polylysine

Pranoprofen (0.01 mol) is conjugated to polylysine (0.01 mol) according to Scheme II omitting the acid washing step. The procedure yields the desired compound ($R_1$ = methyl; $R_2$ = hydrogen; Y = [1]benzopyranol(2,3-b]pyridine; Q = polylysine; m > 50, n/m > 0.2; n > 10).

EXAMPLE 4

Poly ε-Protizinyl Polylysine

Protizinic Acid (0.01 mol) is conjugated to polylysine (0.01 mol-$NH_2$) according to Scheme II omitting the acid washing step. The procedure yields the desired compound ($R_1$ = $CH_3$; $R_2$ = 7-methoxy, 10 dimethyl; Y = phenothiazine; Q = polylysine; m > 50, n/m > 0.2; n > 10).

EXAMPLE 5

Poly ε-Benoxaprofenyl Polylysine

Benoxaprofen (0.01 mol) is conjugated to polylysine (0.01 mol-$NH_2$) according to Scheme II omitting the acid washing step. The procedure yields the desired compound ($R_1$ = methyl; $R_2$ = p-chlorophenyl; Y = benzoxazole; m > 50; Q = polylysine; n/m > 0.2; n > 10).

EXAMPLE 6 [Poly ε-[2-(2-Phenyl-5-Benzoxazolyl) Propionyl] Polylysine

A) 2-(2-Phenyl-5-Benzoxazolyl) Propionic Acid

(i) 2-(4-Hydroxyphenyl) propionitrile

Finely ground 2-(4-aminophenyl) propionitrile (73 g., 0.5 mole) is suspended in concentrated hydrochloric acid (125 ml.). The stirred suspension is diazotised at 0° - 5°C by the dropwise addition of a solution of sodium nitrite (36.23 g., 0.525 mole) in water (60 ml.) during 1-2 hours. The almost clear solution is stirred for a further 20 minutes at 5° - 10°C., then poured into a stirred, boiling solution of concentrated sulphuric acid (250 ml.) in water (2.5 1.). After six minutes, it is cooled in an ice bath, then extracted with ether (x4). The combined other extracts are extracted with 2N-sodium hydroxide solution (x6). The combined alkaline extracts are cooled in an ice bath, acidified with concentrated hydrochloric acid, and extracted with ether (x3). The combined ether extracts are washed with saturated sodium chloride solution (x3), dried ($Na_2SO_4$) and evaporated to leave a dark brown oil (66.7 g.) which on distillation gives 2-(4- hydroxyphenyl) propionitrile (59.58 g.), h.p. 112°C/0.125 mm., m p. 41° - 46°C.

Analysis: Calculated C : 73.44, H : 6.16, N : 9.51. Found C : 73.19, H : 5.91, N : 9.31.

(ii) 2-(3-Nitro-4-hydroxyphenyl)propionitrile

A solution of 2-(4-hydroxyphenyl) propionitrile (7.79 g., 0.053 mole.) in glacial acetic acid (10 ml.) is added at 7° - 10°C., then for 30 minutes at -10°C to -15°C. The suspension is diluted with water (approx. 90 ml.). Filtration yields 2-(3-nitro-4-hydroxyphenyl)propionitrile as a yellow solid (8.43 g.), m.p. 78° - 81°C.

Analysis: Calculated C : 56.24, H : 4.19, N : 14.57. Found C : 56.29, H : 4.24, N : 14.47

(iii)a. 2-(3-Amino-4-hydroxyphenyl) propionitrile

2-(3-Nitro-4-hydroxyphenyl) propionitrile (123.8 g., 0.64 mole.) is suspended in absolute ethanol (950 ml.) and added during 20 minutes, with cooling, to a solution of stannous chloride dihydrate (437.8 g., 1.94 mole.) in concentrated hydrochloric acid (591 ml., 7 mole.). The addition is made at such a rate that the temperature of the reaction mixture did not exceed 20°C. Stirring of the mixture is continued for a further 19 hours at room temperature. The resulting solution, together with ice (1.75 kg.) is added during one hour to a cooled solution of sodium hydroxide (650 g.) in water (600 ml.). The temperature of the reaction mixture is maintained at 15° - 20°C during the addition. The mixture is stirred for a further one hour, and the pH then adjusted to 6 by the addition of concentrated hydrochloric acid. The resulting suspension is filtered, and the filtrate saturated with sodium chloride, then extracted with ether (x6). The combined ether extracts are dried ($Na_2SO_4$) and evaporated to leave a solid (69.15 g.) which is suspended in chloroform and extracted with 2N-hydrochloride acid (x6). The combined acid extracts are neutralized to pH 7-8 by the addition of sodium bicarbonate. The resulting suspension is extracted with ether (x4). The combined ether extracts are washed twice with water, dried ($Na_2SO_4$), and evaporated to yield 2-(3-amino-4-hydroxyphenyl) propionitrile as a light brown solid (62.85 g.), m.p. 110° - 112°C.

Analysis: Calculated C : 66.64, H : 6.21, N : 17.27. Found C : 66.45, H : 6.09, N : 16.99

(iii)b. 2-(3-Amino-4-hydroxyphenyl) propionitrile was also prepared by the following method:

2-)3-Nitro-4-hydroxyphenyl) propionitrile (38.4 g., 0.2 mole.) is suspended in absolute ethanol (250 ml.) and hydrogenated at 4 atmospheres pressure and room temperature over 10% palladium on charcoal. Hydrogenation is complete in 3.8 hours. The catalyst is removed by filtration. Evaporation of the filtrate yields 2-(3-amino-4-hydroxyphenyl) propionitrile (17 g.), m.p. 110°C.

(iv) 2-(3-Benzamido-4-hydroxyphenyl) propionitrile

Benzoyl chloride (27.09 g., 0.19 mole.) is added, with cooling, during 20 minutes to a stirred solution of 2-(3-amino-4-hydroxyphenyl) propionitrile (28.35 g., 0.175 mole.) in dry pyridine (200 ml.) at 0° - 3°C. After addition is complete, the mixture is heated at 100°C. for 1 hour. It is then evaporated under reduced pressure to yield crude 2-(3-benzamido-4-hydroxyphenyl) propionitrile as an oil.

(v) 2-(2-Phenyl-5-benzoxazolyl) propionitrile

The oil from (iv) above is boiled for 30 minutes during or until the temperature of the vapor above the oil rises to 130°C. On cooling, the residue solidifies. Recrystallization of the solid from methanol yields 2-(2-phenyl-5-benzoxazolyl) propionitrile (27.65 g.), m.p. 118° - 120°C.

Analysis: Calculated C : 77.39, H : 4.87, N : 11.28. Found C : 77.23, H : 5.11, N : 11.34

(vi) 2-(2-Phenyl-5-benzoxazolyl) propionic acid

A solution of 2-(2-phenyl-5-benzoxazolyl) propionitrile (24 g., 0.096 mole.) in concentrated hydrochloric acid (220 ml) is refluxed for 2.5 hours. The mixture is poured into ice water (1 liter). The precipitated 2-(2-phenyl-5-benzoxazolyl) propionic acid is filtered off and washed well with water. The dry acid weighs 23 g. and has m.p. 177° - 179°C.

Analysis: Calculated C : 71.89, H : 4.90, N : 5.24. Found C : 72.13, H : 4.95, N : 5.39.

B) Poly ε-[2-(2-phenyl-5-benzoxazoly)propionyl] polylysine

2-(2-phenyl-5-benzoxazolyl) propionic acid (0.01 mol) is conjugated to polylysine (0.01 mol-$NH_2$) according to Scheme II, with the acid washing step omitted. The procedure yields the desired compound ($R_1$ = methyl; $R_2$ = 2-phenyl; Y = 5-benzoxazolyl; m > 50, n/m > 0.2, n > 10.

EXAMPLE 7

Polyvinyl Alcohol Ester of Flunoxaprofen

Flunoxaprofen (0.01 mol) is conjugated to polyvinyl alcohol (0.01 mol-OH) according to Scheme IV. Specifically, thionylchloride is used to prepare the acid chloride of flunoxaprofen. Triethylamine is the base. The procedure yields the desired compound ($R_1 = CH_3$; $R_2$ = 2-(4-fluorophenyl)); Y = benzoxazole; Q = polyvinyl alcohol;

m > 50; n/m > 0.2; n > 10).

EXAMPLE 8

Polynaproxenyl Polyethyleneimine

Naproxen acid chloride was prepared by the procedure of Example 2A. Polyethyleneimine (purity 99% and average molecular weight 1800) 1.7 g was dissolved in 30 ml of pyridine. To this stirred solution was added 4.90 g (0.02 mol) of naproxen acid chloride. The reaction mixture was refluxed for two hours. Concentration in vacuo gave a viscous residue, which was washed with 200 ml of 1 N sodium hydroxide solution. The aqueous part was decanted and the residue oil was washed with five 200 ml portions of water to remove pyridine. The product was dried over phosphorus pentoxide ($P_2O_5$) in a vacuum desiccator at room temperature for 12 days. It gave 3.4 g of polynaproxenyl polyethyleneimine as a yellow solid.

IR (film) 3407, 3296, 3064, 2971, 2936, 1721, 1714, 1667, 1651 $cm^{-1}$.

Elemental analysis calculated for $C_{18}H_{22}N_2O_2 \cdot 1.5\ H_2O$ FW 325;

% Theory: C, 66.46; H, 7.69; N, 8.61. % Found: C, 66.72; H, 7.41; N, 9.21.

The procedure yields the desired compound $R_1$ = methyl; $R_2$ = 6-methoxy; Y = naphthyl; Q = polyethyleneimine; n/m = 0.5.

It will be understood that various changes and modifications can be made in the details of procedure, formulation and use without departing from the spirit of the invention, especially as defined in the following claims.

## Claims

1. A compound of the formula:

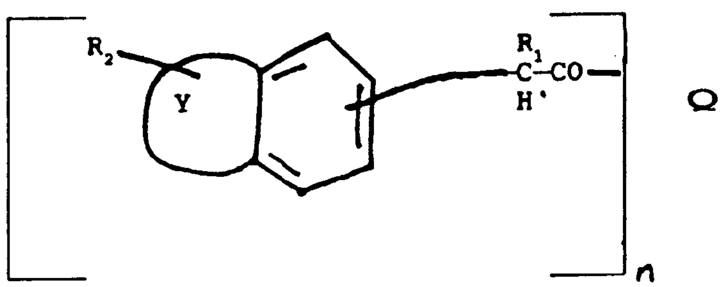

wherein Y, together with the ring to which it is fused forms a multi-ring system selected from napthyl, [1]benzopyranol [2, 3-b], pyridine, phenothiazine, carbazole, and benzoxazole;

$R_1$ is lower alkyl or hydrogen;

$R_2$ is one or more independently selected from cyano, nitro, amino, alkylamino, hydrogen, halogen, lower alkyl, lower alkoxy, hydroxy, phenyl or phenyl substituted with halogen, alkyl or alkoxy;

wherein Q is the deprotonated residue of a polymer or macromolecular structure having a molecular weight of at least 1000 containing at least two primary and/or secondary amino groups and/or hydroxy groups; and

n is an integer of at least 2.

2. A compound as claimed in claim 1 wherein Y forms a naphthyl ring with the ring to which it is fused.

3. A compound as claimed in claim 2 wherein $R_2$ is a lower alkoxy, lower alkyl or halogen.

4. A compound as claimed in claim 3 wherein $R_2$ is a lower alkoxy, lower alkyl or a halogen substituted in the 6-position.

5. A compound as claimed in claim 1 wherein Y forms a [1]benzopyranol[2, 3-b] pyridine ring with the ring to which it is fused.

6. A compound as claimed in claim 6 wherein $R_2$ is hydrogen and $R_1$ is lower alkyl.

7. A compound as claimed in claim 1 wherein Y forms a phenothiazine ring system with the ring to which it is fused.

8. A compound as claimed in claim 7 wherein $R_2$ is lower alkyl substituting the nitrogen on said phenothiazine ring system.

9. A compound as claimed in claim 8 wherein said phenothiazine system is substituted in the 7-position with an alkyl, alkoxy or halogen group.

10. A compound as claimed in claim 9 wherein $R_1$ is lower alkyl.

11. A compound as claimed in claim 9 wherein $R_1$ is hydrogen.

12. A compound as claimed in claim 1 wherein Y forms a 9H-carbazole ring system with the ring to which it is fused.

13. A compound as claimed in claim 12 wherein $R_2$ is a substituent at the 6-position on said carbazole ring system selected from alkyl, alkoxy, or halogen.

14. A compound as claimed in claim 1 wherein Y forms a benzoxazole ring system with the ring to which it is attached.

15. A compound as claimed in claim 14 wherein $R_2$ is a phenyl substituted with halogen, alkyl or alkoxy.

16. A pharmaceutical preparation or composition comprising a compound as claimed in any one of the preceding claims and a pharmaceutically acceptable carrier.

17. Use of a compound as claimed in any one of claims 1 to 15 in the manufacture of a pharmaceutical preparation or composition for the treatment or prevention of colonic polyps.

**Claims for the following Contracting State: ES**

1. A method of manufacturing a compound for treating colonic polyps comprising conjugating a compound of formula I

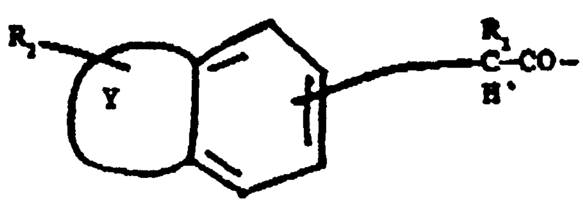

I

wherein Y, together with the ring to which it is fused forms a multi-ring system selected from the group consisting of napthyl, [1]benzopyranol [2, 3-b], pyridine, phenothiazine, carbazole, and benzoxazole;

$R_1$ is lower alkyl or hydrogen; and

$R_2$ is one or more independly selected from cyano, nitro, amino, alkylamino, hydrogen, halogen, lower alkyl, lower alkoxy, hydroxy, phenyl or phenyl substituted with halogen, alkyl or alkoxy;

with a deprotonated residue of a polymer or macromolecular structure having a molecular weight of at least 1000 containing at least two primary and/or secondary amino groups and/or hydroxy groups, wherein at least two moles of the compound of the formula I are conjugated to said polymer or macromolecule.

2. A method according to claim 1 wherein Y forms a naphthyl ring with the ring to which it is fused.

3. A method according to claim 2 wherein $R_2$ is a lower alkoxy, lower alkyl or halogen.

4. A method according to claim 3 wherein $R_2$ is a lower alkoxy, lower alkyl or a halogen substituted in the 6-position.

5. A method according to claim 3 wherein $R_1$ is lower alkyl.

6. A method according to claim 1 wherein Y forms a [1]benzopyranol[2, 3-b] pyridine ring with the ring to which it is fused.

7. A method according to claim 6 wherein $R_2$ is hydrogen and $R_1$ is lower alkyl.

8. A method according to claim 1 wherein Y forms a phenothiazine ring system with the ring to which it is fused.

9. A method according to claim 8 wherein $R_2$ is lower alkyl substituting the nitrogen on said phenothiazine ring system.

10. A method according to claim 9 wherein said phenothiazine system is substituted in the 7-position with an alkyl, alkoxy or halogen group.

11. A method according to claim 10 wherein $R_1$ is lower alkyl.

12. A method according to claim 10 wherein $R_1$ is hydrogen.

13. A method according to claim 1 wherein Y forms a 9H-carbazole ring system with the ring to which it is fused.

14. A method according to claim 13 wherein $R_2$ is a substituent at the 6-position on said carbazole ring system selected from alkyl, alkoxy, or halogen.

15. A method according to claim 1 wherein Y forms a benzoxazole ring system with the ring to which it is attached.

16. A method according to claim 15 wherein $R_2$ is a phenyl substituted with halogen, alkyl or alkoxy.